# EUROPEAN PATENT APPLICATION

(11) **EP 2 025 353 A2**
(43) Date of publication of application: **18.02.2009**
(21) Application number: 08075891.5
(22) Date of filing: 29.04.1994
(51) Int. Cl.: A61L 27/20, A61L 27/38, A61L 27/50

(54) **Injectable polysaccharide-cell compositions**

(30) Priority: 30.04.1993 US 56140; 18.04.1994 US 228678; 18.04.1994 US 229464
(62) Divisional of application: 94919101.9
(71) Applicant: Massachusetts Institute of Technology, Cambridge, MA 02139 (US); The Children's Medical Center Corporation, Boston, Massachusetts 02115 (US)
(72) Inventor: Griffith-Cima, Linda, Lexington Massachusetts 02173 (US); Atala, Anthony, Newton Massachusetts 02167 (US); Vacanti, Charles A., Lexington Massachusetts 02173 (US); Paige, Keith T., Brookline Massachusetts 02146 (US)
(74) Representative: Wright, Andrew John

(57) **Abstract**

Slowly polymerizing polysaccharide hydrogels have been demonstrated to be useful as a means of delivering large numbers of isolated cells via injection. The gels promote engraftment and provide three dimensional templates for new cell growth. The resulting tissue is similar in composition and histology to naturally occurring tissue. This method can be used for a variety of reconstructive procedures, including custom molding of cell implants to reconstruct three dimensional tissue defects, as well as implantation of tissues generally. Examples demonstrate construction of muscle and cartilage tissues which are useful to repair defects such as reflux and incontinence.

## Description

### Background of the Invention

The present invention is generally in the area of using polysaccharide hydrogel-cell compositions in the area of medical treatments, and specifically relates to a method for correcting vesicoureteral reflux, incontinence and other defects.

### Craniofacial contour deformities

Craniofacial contour deformities, whether traumatic, congenital, or aesthetic, currently require invasive surgical techniques for correction. Furthermore, deformities requiring augmentation often necessitate the use of alloplastic prostheses which suffer from problems of infection and extrusion. A minimally invasive method of delivering additional autogenous cartilage or bone to the craniofacial skeleton would minimize surgical trauma and eliminate the need for alloplastic prostheses. If one could transplant via injection and cause to engraft large numbers of isolated cells, one could augment the craniofacial osteo-cartilaginous skeleton with autogenous tissue, but without extensive surgery.

Unfortunately, attempts to inject dissociated cells subcutaneously or to implant dissociated tissues within areas of the body such as the peritoneum have not been successful. Cells are relatively quickly removed, presumably by phagocytosis and cell death.

Cells can be implanted onto a polymeric matrix and implanted to form a cartilaginous structure, as described in U.S. Patent No. 5,041,138 to Vacanti, et al., but this requires surgical implantation of the matrix and shaping of the matrix prior to implantation to form a desired anatomical structure.

### Vesicoureteral reflux.

Vesicoureteral reflux is a condition wherein there is an abnormal development of the ureteral bud as it enters the bladder during embryologic development. The shortened course of the ureter through the bladder musculature decreases the ureteral resistance and allows for urine to reflux from the bladder reservoir back up into the ureter and into the kidney. With this condition, bacteria which may occasionally be present in the bladder through retrograde urethral transport, can reach the kidneys and cause recurrent pyelonephritis. In addition, the constant back pressure of the urine into the calyces and renal pyramids results in mechanical damage to the renal parenchyma. If untreated, urinary vesicoureteral reflux can cause loss of renal parenchyma, and in some instances, renal failure, as reviewed by Atala and Casale, Infections in Urology 39-43 (March/April 1990). In 1960, 70% of the patients with renal failure were described as having vesicoureteral reflux as the primary etiology. With the advent of new diagnostic and treatment modalities, patients with vesicoureteral reflux now account for less than 1% of the renal failure population.

In the past, vesicoureteral reflux was usually diagnosed with a voiding cystogram after the child presented with repeated episodes of pyelonephritis. With the increased use of prenatal and postnatal sonography, hydronephrosis is more detectable, prompting further radiologic workup and earlier detection, as reported by Atala and Casale. Vesicoureteral reflux is graded depending on the severity. Grade 1 reflux signifies that urine is seen refluxing from the bladder up to the ureter only; in grade 2 reflux, urine refluxes into the ureter and calyceal dilatation. Grade 4 and 5 reflux are more severe, showing ureteral tortuosity and further calyceal blunting and dilatation, respectively.

The treatment of vesicoureteral reflux has been well established over the last decade. Initially it was believed that all patients with reflux would require surgery. Another school of management soon proposed that only medical therapy with antibiotics was required. It is now well established that the treatment of reflux depends on many factors, including the severity of reflux, associated congenital abnormalities, and the social situation of the child (parental compliance with medical treatment). Medical treatment is usually recommended for patients with grade 1 and 2 reflux, which usually resolve on their own as the bladder/ureteral configuration changes with growth. Grade 3 reflux is generally managed with medical therapy unless it persists or breakthrough infections occur while on antibiotic suppression. Surgical treatment is usually required for grade 4 and 5 reflux.

Medical treatment implies that the patient is treated with daily suppressive antibiotics. A close follow-up is required in these patients, generally consisting of a catheterized urine culture every three months, an ultrasound exam and serum analysis every six months, a fluoroscopic or nuclear voiding cystourethrogram every year, and a DMSA renal scan every two years. Surgical treatment consists of an open surgery wherein a low abdominal incision is made, the bladder is entered, the ureters are mobilized and new ureteral submucosal tunnels are created; thereby extending the muscular backing of the ureter which increases their resistance. These patients require a general endotracheal anesthetic for a four to five hour surgery, an epidural catheter for both intraoperative and postoperative pain control, a bladder catheter for drainage, a perivesical drain, and a five to six day hospital stay. Antibiotic therapy and bladder antispasmodics are required post-operatively.

Although open surgical procedures for the correction of reflux have excellent results in the hands of experienced surgeons, it is associated with a well recognized morbidity, including pain and immobilization of a lower abdominal incision, bladder spasms, hematuria, and post-operative voiding frequency in some children. In an effort to avoid open surgical intervention, widespread interest was initiated by Matouschek's clinical experience with the endoscopic injection of Teflon^{™} (polytetrafluoroethylene) paste subureterally in 1984, as reported in Matouschek, E.: Die Behandlung des vesikorenalen Refluxes durch transueterale Einspritzung von polytetrafluoroethylenepast. Urologe, 20:263 (1981). With this technique, a cystoscope is inserted into the bladders, a needle is inserted through the cystoscope and placed under direct vision underneath the refluxing ureter in the submucosal space, and Teflon^{™} paste is injected until the gaping ureteric orifice configuration changes into a half-moon slit. The Teflon^{™} paste, injected endoscopically, corrects the reflux by acting as a bulking material which increases ureteral resistance. However, soon after the introduction of this treatment, a controversy regarding the use of Teflon^{™} paste ensued. Malizia et al. "Migration and granulomatous reaction after periurethral injection of polymer (polytetrafluoroethylene)" JAMA, 251:3277 (1984), showed granuloma formation and particulate migration to the brain, lungs, and lymph nodes in animal studies. Polytetrafluoroethylene migration and granuloma formation have also been reported in humans by Claes et al., "Pulmonary migration following periurethral polytetrafluoroethylene injection for urinary incontinence" J. Urol., 142:821 (1989). The safety of Teflon^{™} for human use was questioned, and the paste was thereafter banned by the FDA.

However, there are definite advantages in treating vesicoureteral reflux endoscopically. The method is simple and can be completed in less than fifteen minutes, it has a success rate of greater than 85% with low morbidity and it can be performed in an outpatient basis, as reported by Atala et al, "Endoscopic treatment of vesicoureteral reflux with a self-detachable balloon system" J. Urol. 148:724 (1992). The goal of several investigators has been to find alternate implant materials which would be safe for human use.

Bovine dermal collagen preparations have been used to treat reflux endoscopically. However, only 58.5% of the patients were cured at one year follow-up, as described by Leonard et al, "Endoscopic injection of glutaraldehyde cross-linked bovine dermal collagen for correction of vesicoureteral reflux" J. Urol. 145:115 (1991). The collagen implant volume decreases with time, which results in high percentage of recurrence of reflux, over 90% within 3 years. The high failure rate with this substance presents a high risk to the unaware patient of developing renal damage after treatment.

A paste consisting of textured microparticles of silicone, suspended in a hydrogel, has been injected subureterally to correct reflux with an initial success rate of 91% in one European study, as reported by Buckley et al., "Endoscopic correction of vesicoureteric reflux with injectable silicone microparticles" J. Urol. 149: 259A (1993). However, distant particle migration has been observed in animal models, as reported by Henly et al., "Particulate silicone for use in periurethral injections: a study of local tissue effects and a search for migration" J. Urol. 147:376A (1992). Approximately thirty percent of the silicone particles have a diameter which is less than 100 µm. This suggests that thirty percent of the silicone particles have a potential for distant organ migration through the macrophage system. The manufacturer of this technology tried unsuccessfully to obtain FDA approval, and subsequently filed for bankruptcy.

Laparoscopic correction of reflux has been attempted in both an animal model (Atala et al, "Laparoscopic correction of vesicoureteral reflux" J. Urol. 150:748 (1993)) and humans (Atala, "Laparoscopic treatment of vesicoureteral reflux" Dial. Ped. Urol. 14:212 (1993)) and is technically feasible. However, at least two surgeons with laparoscopic expertise are needed, the length of the procedure is much longer than with open surgery, the surgery is converted from an extraperitoneal to an intraperitoneal approach, and the cost is higher due to both increased operative time and the expense of the disposable laparoscopic equipment.

Despite the fact that over a decade has transpired since the Teflon^{™} controversy, little progress has been made in this area of research. The ideal substance for the endoscopic treatment of reflux should be injectable, non-antigenic, non-migratory, volume stable, and safe for human use (Atala et al, 1992).

### Urinary incontinence.

Urinary Incontinence is the most common and the most intractable of all GU maladies. Urinary incontinence, or the inability to retain urine and not void urine involuntarily, is dependent on the interaction of two sets of muscles. One is the detrusor muscle, a complex of longitudinal fibers forming the external muscular coating of the bladder. The detrusor is activated by parasympathetic nerves. The second muscle is the smooth/striated muscle of the bladder sphincter. The act of voiding requires the sphincter muscle be voluntarily relaxed at the same time that the detrusor muscle of the bladder contracts. As a person ages, his ability to voluntarily control the sphincter muscle is lost in the same way that general muscle tone deteriorates with age. This can also occur when a radical event such as paraplegia "disconnects" the parasympathetic nervous system causing a loss of sphincter control. In different patients, urinary incontinence exhibits different levels of severity and is classified accordingly.

The most common incontinence, particular in the elderly, is urge incontinence. This type of incontinence is characterized by an extremely brief warning following by immediate urination. This type of incontinence is caused by a hyperactive detrusor and is usually treated with "toilet training" or medication. Reflex incontinence, on the other hand, exhibits no warning and is usually the result of an impairment of the parasympathetic nerve system such as a spinal cord injury.

Stress incontinence is most common in elderly women but can be found in women of any age. It is also commonly seen in pregnant women. This type of incontinence accounts for over half of the total number of cases. It is also found in men but at a lower incidence. Stress incontinence is characterized by urine leaking under conditions of stress such as sneezing, laughing or physical effort. There are five recognized categories of severity of stress incontinence, designated as types as 0, 1, 2a, 2b, and 3. Type 3 is the most severe and requires a diagnosis of intrinsic Sphincter Deficiency or ISD (Contemporary Urology, March 1993). There are many popular treatments including weight loss, exercise, medication and in more extreme cases, surgical intervention. The two most common surgical procedures involve either elevating the bladder neck to counteract leakage or constructing a lining from the patient's own body tissue or a prosthetic material such as PTFE to put pressure on the urethra. Another option is to use prosthetic devices such as artificial sphincters to external devices such as intravaginal balloons or penile clamps. For treatment of type 3 stress incontinence, there has been a recent trend toward injection of Teflon^{™} or collagen paste around the sphincter muscle in order to "beef up" the area and improve muscle tone. None of the above methods of treatment, however, are very effective for periods in excess of a year.

Overflow incontinence is caused by anatomical obstructions in the bladder or underactive detrustors. It is characterized by a distended bladder which leads to frequent urine leakage. This type of incontinence is treated acutely by catheterization and long-term by drug therapy. Enuresis or bed-wetting is a problem in pediatrics and is controlled by various alarming devices and pads with sensors. Enuresis is not considered a serious problem unless it lasts beyond the age of four or five. Finally, there is true functional incontinence which occurs in patients with chronic impairment either of mobility or mental function. Such patients are usually treated by the use of diapers, incontinence pads or continuous catheterization (BBI, 1985 Report 7062).

Accordingly, it is an object of the present invention to provide a method and compositions for injection of cells to form cellular tissues and cartilaginous structures.

It is a further object of the invention to provide compositions to form cellular tissues and cartilaginous structures including non-cellular material which will degrade and be removed to leave tissue or cartilage that is histologically and chemically the same as naturally produced tissue or cartilage.

It is another object of the present invention to provide a method and material for treating vesicoureteral reflux, incontinence, and other defects which results in a natural and permanent cure to the defect.

It is a further object of the present invention to provide a method and material for treating vesicoureteral reflux, incontinence, and other defects which is quick, simple, safe, and relatively non-invasive.

### Summary of the Invention

Slowly polymerizing, biocompatible, biodegradable hydrogels have been demonstrated to be useful as a means of delivering large numbers of isolated cells into a patient to create an organ equivalent or tissue such as cartilage. The gels promote engraftment and provide three dimensional templates for new cell growth. The resulting tissue is similar in composition and histology to naturally occurring tissue. In one embodiment, cells are suspended in a hydrogel solution and injected directly into a site in a patient, where the hydrogel hardens into a matrix having cells dispersed therein. In a second embodiment, cells are suspended in a hydrogel solution which is poured or injected into a mold having a desired anatomical shape, then hardened to form a matrix having cells dispersed therein which can be implanted into a patient. Ultimately, the hydrogel degrades, leaving only the resulting tissue.

This method can be used for a variety of reconstructive procedures, including custom molding of cell implants to reconstruct three dimensional tissue defects, as well as implantation of tissues generally.

A method of treatment of vesicoureteral reflux, incontinence and other defects is described wherein bladder muscle cells are mixed with a liquid polymeric material, to form a cell suspension, which is injected into the area of the defect, in an amount effective to yield a tissue that corrects the defect, for example, which provides the required control over the passage of urine. As described in the examples, human bladder muscle specimens or chondrocytes are obtained and processed, the cells are mixed with alginate, which is designed to solidify at a controlled rate when contacted with calcium salts, and the cells are then injected at the desired site where they proliferate and correct the defect. Examples demonstrate efficacy in mice and pigs.

### Brief Description of the Drawings

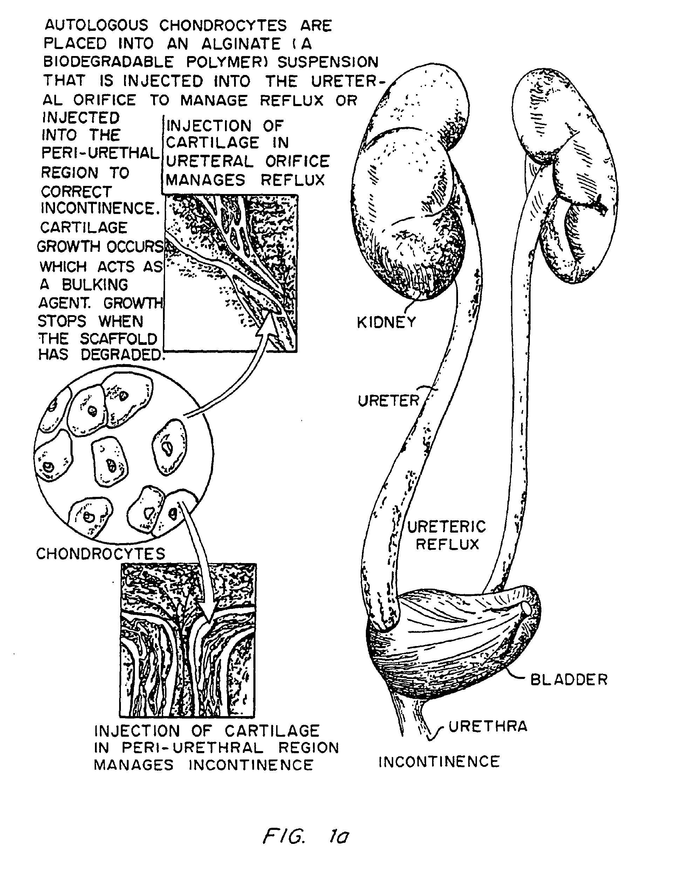

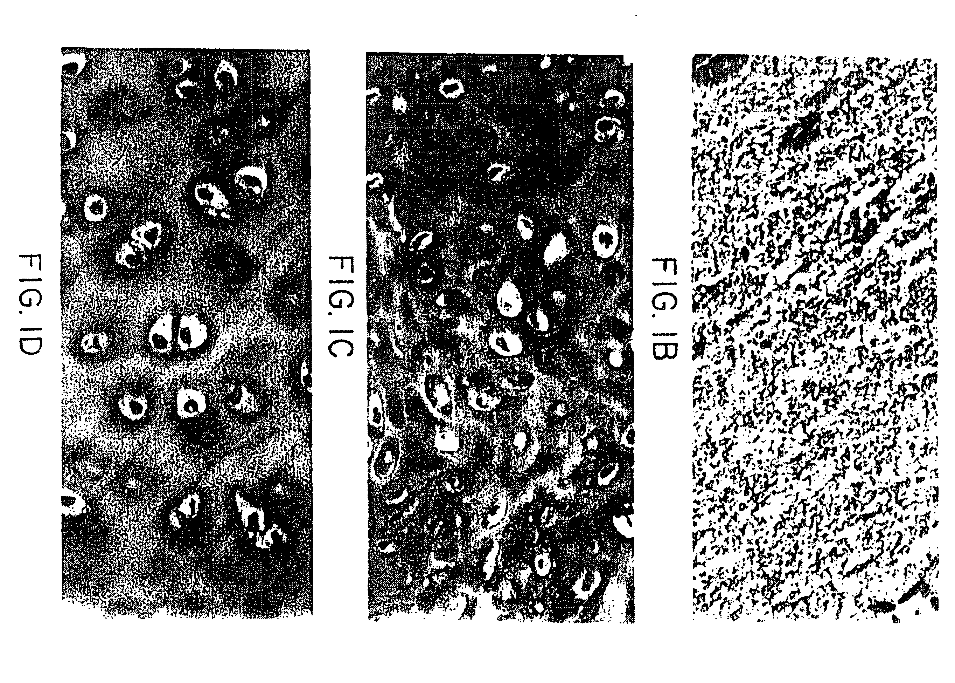

Figures 1a-1d are a schematic of injection of a polymer-chondrocyte suspension into a region of the ureteral orifice for control of vesicoureteral reflux or into the peri-urethral region to manage incontinence; with photomicrographs showing the alginate polymer suspension, new cartilage formed following injection of the suspension, and a cartilage cell control injected in the absence of polymer.

### Detailed Description of the Invention

Techniques of tissue engineering employing biocompatible polymer scaffolds hold promise as a means of creating alternatives to prosthetic materials currently used in craniomaxillofacial surgery, as well as formation of organ equivalents to replaced diseased, defective, or injured tissues. However, polymers used to create these scaffolds, such as polylactic acid, polyorthoesters, and polyanhydrides, are difficult to mold and hydrophobic, resulting in poor cell attachment. Moreover, all manipulations of the polymers must be performed prior to implantation of the polymeric material.

Calcium alginate and certain other polymers can form ionic hydrogels which are malleable and can be used to encapsulate cells. In the preferred embodiment described herein, the hydrogel is produced by cross-linking the anionic salt of alginic acid, a carbohydrate polymer isolated from seaweed, with calcium cations, whose strength increases with either increasing concentrations of calcium ions or alginate. The alginate solution is mixed with the cells to be implanted to form an alginate suspension. Then, in one embodiment, the suspension is injected directly into a patient prior to hardening of the suspension. The suspension then hardens over a short period of time. In a second embodiment, the suspension is injected or poured into a mold, where it hardens to form a desired anatomical shape having cells dispersed therein.

### Source of Cells

Cells can be obtained directed from a donor, from cell culture of cells from a donor, or from established cell culture lines. In the preferred embodiment, cells of the same species and preferably immunological profile are obtained by biopsy, either from the patient or a close relative, which are then grown to confluence in culture using standard conditions, for example, as described below in Example 1 and used as needed. If cells that are likely to elicit an immune reaction are used, such as human muscle cells from immunologically distinct individual, then the recipient can be immunosuppressed as needed, for example, using a schedule of steroids and other immunosuppressant drugs such as cyclosporine. However, in the most preferred embodiment, the cells are autologous.

In the preferred embodiments, cells are obtained directly from a donor, washed and implanted directly in combination with the polymeric material. The cells are cultured using techniques known to those skilled in the art of tissue culture.

Cells obtained by biopsy are harvested and cultured, passaging as necessary to remove contaminating cells. Isolation of chondrocytes and muscle cells are demonstrated in the examples.

Cell attachment and viability can be assessed using scanning electron microscopy, histology, and quantitative assessment with radioisotopes. The function of the implanted cells can be determined using a combination of the above-techniques and functional assays. For example, in the case of hepatocytes, in vivo liver function studies can be performed by placing a cannula into the recipient's common bile duct. Bile can then be collected in increments. Bile pigments can be analyzed by high pressure liquid chromatography looking for underivatized tetrapyrroles or by thin layer chromatography after being converted to azodipyrroles by reaction with diazotized azodipyrroles ethylanthranilate either with or without treatment with P-glucuronidase. Diconjugated and monoconjugated bilirubin can also be determined by thin layer chromatography after alkalinemethanolysis of conjugated bile pigments. In general, as the number of functioning transplanted hepatocytes increases, the levels of conjugated bilirubin will increase. Simple liver function tests can also be done on blood samples, such as albumin production. Analogous organ function studies can be conducted using techniques known to those skilled in the art, as required to determine the extent of cell function after implantation. For example, islet cells of the pancreas may be delivered in a similar fashion to that specifically used to implant hepatocytes, to achieve glucose regulation by appropriate secretion of insulin to cure diabetes. Other endocrine tissues can also be implanted. Studies using labelled glucose as well as studies using protein assays can be performed to quantitate cell mass on the polymer scaffolds. These studies of cell mass can then be correlated with cell functional studies to determine what the appropriate cell mass is. In the case of chondrocytes, function is defined as providing appropriate structural support for the surrounding attached tissues.

This technique can be used to provide multiple cell types, including genetically altered cells, within a three-dimensional scaffolding for the efficient transfer of large number of cells and the promotion of transplant engraftment for the purpose of creating a new tissue or tissue equivalent. It can also be used for immunoprotection of cell transplants while a new tissue or tissue equivalent is growing by excluding the host immune system.

Examples of cells which can be implanted as described herein include chondrocytes and other cells that form cartilage, osteoblasts and other cells that form bone, muscle cells, fibroblasts, and organ cells. As used herein, "organ cells" includes hepatocytes, islet cells, cells of intestinal origin, cells derived from the kidney, and other cells acting primarily to synthesize and secret, or to metabolize materials.

### Addition of Biologically Active Materials to the hydrogel.

The polymeric matrix can be combined with humoral factors to promote cell transplantation and engraftment. For example, the polymeric matrix can be combined with angiogenic factors, antibiotics, antiinflammatories, growth factors, compounds which induce differentiation, and other factors which are known to those skilled in the art of cell culture.

For example, humoral factors could be mixed in a slow-release form with the cell-alginate suspension prior to formation of implant or transplantation. Alternatively, the hydrogel could be modified to bind humoral factors or signal recognition sequences prior to combination with isolated cell suspension.

### Polymer Solutions

In the preferred embodiment described herein, calcium alginate and certain other polymers that can form ionic hydrogels which are malleable are used to encapsulate cells. The hydrogel is produced by cross-linking the anionic salt of alginic acid, a carbohydrate polymer isolated from seaweed, with calcium cations, whose strength increases with either increasing concentrations of calcium ions or alginate. The alginate solution is mixed with the cells to be implanted to form an alginate suspension. Then the suspension is injected directly into a patient prior to hardening of the suspension. The suspension then hardens over a short period of time due to the presence in vivo of physiological concentrations of calcium ions.

The polymeric material which is mixed with cells for implantation into the body should form a hydrogel. A hydrogel is defined as a substance formed when an organic polymer (natural or synthetic) is cross-linked via covalent, ionic, or hydrogen bonds to create a three-dimensional open-lattice structure which entraps water molecules to form a gel. Examples of materials which can be used to form a hydrogel include polysaccharides such as alginate, polyphosphazines, and polyacrylates, which are crosslinked ionically, or block copolymers such as Pluronics^{™} or Tetronics^{™}, polyethylene oxide-polypropylene glycol block copolymers which are crosslinked by temperature or pH, respectively. Other materials include proteins such as fibrin, polymers such as polyvinylpyrrolidone, hyaluronic acid and collagen.

In general, these polymers are at least partially soluble in aqueous solutions, such as water, buffered salt solutions, or aqueous alcohol solutions, that have charged side groups, or a monovalent ionic salt thereof. Examples of polymers with acidic side groups that can be reacted with cations are poly(phosphazenes), poly(acrylic acids), poly(methacrylic acids), copolymers of acrylic acid and methacrylic acid, poly(vinyl acetate), and sulfonated polymers, such as sulfonated polystyrene. Copolymers having acidic side groups formed by reaction of acrylic or methacrylic acid and vinyl ether monomers or polymers can also be used. Examples of acidic groups are carboxylic acid groups, sulfonic acid groups, halogenated (preferably fluorinated) alcohol groups, phenolic OH groups, and acidic OH groups.

Examples of polymers with basic side groups that can be reacted with anions are poly(vinyl amines), poly(vinyl pyridine), poly(vinyl imidazole), and some imino substituted polyphosphazenes. The ammonium or quaternary salt of the polymers can also be formed from the backbone nitrogens or pendant imino groups. Examples of basic side groups are amino and imino groups.

Alginate can be ionically cross-linked with divalent cations, in water, at room temperature, to form a hydrogel matrix. Due to these mild conditions, alginate has been the most commonly used polymer for hybridoma cell encapsulation, as described, for example, in U.S. Patent No. 4,352,883 to Lim. In the Lim process, an aqueous solution containing the biological materials to be encapsulated is suspended in a solution of a water soluble polymer, the suspension is formed into droplets which are configured into discrete microcapsules by contact with multivalent cations, then the surface of the microcapsules is crosslinked with polyamino acids to form a semipermeable membrane around the encapsulated materials.

Polyphosphazenes are polymers with backbones consisting of nitrogen and phosphorous separated by alternating single and double bonds. Each phosphorous atom is covalently bonded to two side chains ("R"). The repeat unit in polyphosphazenes has the general structure (1): 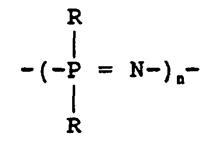 where n is an integer.

The polyphosphazenes suitable for cross-linking have a majority of side chain groups which are acidic and capable of forming salt bridges with di- or trivalent cations. Examples of preferred acidic side groups are carboxylic acid groups and sulfonic acid groups. Hydrolytically stable polyphosphazenes are formed of monomers having carboxylic acid side groups that are crosslinked by divalent or trivalent cations such as Ca²⁺ or Al³⁺. Polymers can be synthesized that degrade by hydrolysis by incorporating monomers having imidazole, amino acid ester, or glycerol side groups. For example, a polyanionic poly[bis(carboxylatophenoxy)]phosphazene (PCPP) can be synthesized, which is cross-linked with dissolved multivalent cations in aqueous media at room temperature or below to form hydrogel matrices.

Bioerodible polyphosphazines have at least two differing types of side chains, acidic side groups capable of forming salt bridges with multivalent cations, and side groups that hydrolyze under *in vivo* conditions, e.g., imidazole groups, amino acid esters, glycerol and glucosyl. The term bioerodible or biodegradable, as used herein, means a polymer that dissolves or degrades within a period that is acceptable in the desired application (usually *in vivo* therapy), less than about five years and most preferably less than about one year, once exposed to a physiological solution of pH 6-8 having a temperature of between about 25°C and 38°C. Hydrolysis of the side chain results in erosion of the polymer. Examples of hydrolyzing side chains are unsubstituted and substituted imidizoles and amino acid esters in which the group is bonded to the phosphorous atom through an amino linkage (polyphosphazene polymers in which both R groups are attached in this manner are known as polyaminophosphazenes). For polyimidazolephosphazenes, some of the "R" groups on the polyphosphazene backbone are imidazole rings, attached to phosphorous in the backbone through a ring nitrogen atom. Other "R" groups can be organic residues that do not participate in hydrolysis, such as methyl phenoxy groups or other groups shown in the scientific paper of Allcock, et al., Macromolecule 10:824-830 (1977).

Methods for synthesis and the analysis of various types of polyphosphazenes are described by Allcock, H.R.; et al., Inorg. Chem. 11, 2584 (1972); Allcock, et al., Macromolecules 16, 715 (1983); Allcock, et al., Macromolecules 19, 1508 (1986); Allcock, et al., Biomaterials, 19, 500 (1988); Allcock, et al., Macromolecules 21, 1980 (1988); Allcock, et al., Inorg. Chem. 21(2), 515-521 (1982); Allcock, et al., Macromolecules 22, 75 (1989); U.S. Patent Nos. 4,440,921, 4,495,174 and 4,880,622 to Allcock, et al.; U.S. Patent No. 4,946,938 to Magill, et al.; and Grolleman, et al., J. Controlled Release 3, 143 (1986), the teachings of which are specifically incorporated herein by reference.

Methods for the synthesis of the other polymers described above are known to those skilled in the art. See, for example Concise Encyclopedia of Polymer Science and Polymeric Amines and Ammonium Salts, E. Goethals, editor (Pergamen Press, Elmsford, NY 1980). Many polymers, such as poly(acrylic acid), are commercially available.

The water soluble polymer with charged side groups is crosslinked by reacting the polymer with an aqueous solution containing multivalent ions of the opposite charge, either multivalent cations if the polymer has acidic side groups or multivalent anions if the polymer has basic side groups. The preferred cations for cross-linking of the polymers with acidic side groups to form a hydrogel are divalent and trivalent cations such as copper, calcium, aluminum, magnesium, strontium, barium, and tin, although di-, tri- or tetra-functional organic cations such as alkylammonium salts, e.g., R₃N⁺ -\/\/\/-⁺NR₃ can also be used. Aqueous solutions of the salts of these cations are added to the polymers to form soft, highly swollen hydrogels and membranes. The higher the concentration of cation, or the higher the valence, the greater the degree of cross-linking of the polymer. Concentrations from as low as 0.005 M have been demonstrated to cross-link the polymer. Higher concentrations are limited by the solubility of the salt.

The preferred anions for cross-linking of the polymers to form a hydrogel are divalent and trivalent anions such as low molecular weight dicarboxylic acids, for example, terepthalic acid, sulfate ions and carbonate ions. Aqueous solutions of the salts of these anions are added to the polymers to form soft, highly swollen hydrogels and membranes, as described with respect to cations.

A variety of polycations can be used to complex and thereby stabilize the polymer hydrogel into a semi-permeable surface membrane. Examples of materials that can be used include polymers having basic reactive groups such as amine or imine groups, having a preferred molecular weight between 3,000 and 100,000, such as polyethylenimine and polylysine. These are commercially available. One polycation is poly(L-lysine); examples of synthetic polyamines are: polyethyleneimine, poly(vinylamine), and poly(allyl amine). There are also natural polycations such as the polysaccharide, chitosan.

Polyanions that can be used to form a semi-permeable membrane by reaction with basic surface groups on the polymer hydrogel include polymers and copolymers of acrylic acid, methacrylic acid, and other derivatives of acrylic acid, polymers with pendant SO₃H groups such as sulfonated polystyrene, and polystyrene with carboxylic acid groups.

### Cell Suspensions

Preferably the polymer is dissolved in an aqueous solution, preferably a 0.1 M potassium phosphate solution, at physiological pH, to a concentration forming a polymeric hydrogel, for example, for alginate, of between 0.5 to 2% by weight, preferably 1%, alginate. The isolated cells are suspended in the polymer solution to a concentration of between 1 and 50 million cells/ml, most preferably between 10 and 20 million cells/ml.

### Methods of Implantation.

The techniques described herein can be used for delivery of many different cell types to achieve different tissue structures. In the preferred embodiment, the cells are mixed with the hydrogel solution and injected directly into a site where it is desired to implant the cells, prior to hardening of the hydrogel. However, the matrix may also be molded and implanted in one or more different areas of the body to suit a particular application. This application is particularly relevant where a specific structural design is desired or where the area into which the cells are to be implanted lacks specific structure or support to facilitate growth and proliferation of the cells.

The site, or sites, where cells are to be implanted is determined based on individual need, as is the requisite number of cells. For cells having organ function, for example, hepatocytes or islet cells, the mixture can be injected into the mesentery, subcutaneous tissue, retroperitoneum, properitoneal space, and intramuscular space. For formation of cartilage, the cells are injected into the site where cartilage formation is desired. One could also apply an external mold to shape the injected solution. Additionally, by controlling the rate of polymerization, it is possible to mold the cell-hydrogel injected implant like one would mold clay.

Alternatively, the mixture can be injected into a mold, the hydrogel allowed to harden, then the material implanted.

The suspension can be injected via a syringe and needle directly into a specific area wherever a bulking agent is desired, i.e., a soft tissue deformity such as that seen with areas of muscle atrophy due to congenital or acquired diseases or secondary to trauma, burns, and the like. An example of this would be the injection of the suspension in the upper torso of a patient with muscular atrophy secondary to nerve damage.

The suspension can also be injected as a bulking agent for hard tissue defects, such as bone or cartilage defects, either congenital or acquired disease states, or secondary to trauma, burns, or the like. An example of this would be an injection into the area surrounding the skull where a bony deformity exists secondary to trauma. The injunction in these instances can be made directly into the needed area with the use of a needle and syringe under local or general anesthesia.

The suspension could also be injected percutaneously by direct palpation, such as by placing a needle inside the vas deferens and occluding the same with the injected bulking substance, thus rendering the patient infertile. The suspension could also be injected through a catheter or needle with fluoroscopic, sonographic, computed tomography, magnetic resonance imaging or other type of radiologic guidance. This would allow for placement or injection of this substance either by vascular access or percutaneous access to specific organs or other tissue regions in the body, wherever a bulking agent would be required.

Further, this substance could be injected through a laparoscopic or thoracoscope to any intraperitoneal or extraperitoneal or thoracic organ. For example, the suspension could be injected in the region of the gastro-esophageal junction for the correcting of gastroesophageal reflux. This could be performed either with a thoracoscope injecting the substance in the esophageal portion of the gastroesophageal region, or via a laparoscope by injecting the substance in the gastric portion of the gastroesophageal region, or by a combined approach.

Vesicoureteral reflux is one of the most common congenital defects in children, affecting approximately 1% of the population. Although all patients do not require surgical treatment, it is still one of the most common procedure performed in children. Over 600 ureteral reimplants are performed yearly at Children's Hospital in Boston, Massachusetts. This translates to an approximately saving of 3600 inpatient hospital days per year at this institution alone, if the endoscopic treatment described herein is used instead of open surgery.

In addition to its use for the endoscopic treatment of reflux, the system of injectable autologous muscle cell may also be applicable for the treatment of other medical conditions, such as urinary and rectal incontinence, dysphonia, plastic reconstruction, and wherever an injectable permanent biocompatible material is needed.

As described herein, an injectable biodegradable polymer as a delivery vehicle for muscle cells or chondrocytes is useful in the treatment of reflux and incontinence. In the preferred embodiment, a biopsy is obtained under anesthesia from a patient with vesicoureteral reflux, the isolated cells are mixed with alginate, and the cell-alginate solution is injected endoscopically in the sub-ureteral region to correct reflux, as shown in Figure la. The time to solidification of the alginate-cell solution may be manipulated by varying the concentration of calcium as well as the temperature at which the cells are added to the alginate. The use of autologous cells precludes an immunologic reaction. Solidification of the alginate impedes its migration until after it is degraded. The suspension can be injected through a cystoscopic needle, having direct visual access with a cystoscope to the area of interest, such as for the treatment of vesico-ureteral reflux or urinary incontinence. In addition to the use of the cell-polymer suspension for the treatment of reflux and incontinence, the suspension can also be applied to reconstructive surgery, as well as its application anywhere in the human body where a biocompatible permanent injectable material is necessary. The suspension can be injected endoscopically, for example through a laryngoscope for injection into the vocal chords for the treatment of dysphonia, or through a hysteroscope for injection into the fallopian tubes as a method of rendering the patient infertile, or through a proctoscope, for injection of the substance in the perirectal sphincter area, thereby increasing the resistance in the sphincter area and rendering the patient continent of stool.

This technology can be used for other purposes. For example, custom-molded cell implants can be used to reconstruct three dimensional tissue defects, e.g., molds of human ears could be created and a chondrocyte-hydrogel replica could be fashioned and implanted to reconstruct a missing ear. Cells can also be transplanted in the form of a thee-dimensional structure which could be delivered via injection.

The present invention will be further understood by reference to the following nonlimiting examples.

### Example 1: Preparation of a Calcium-Alginatechondrocyte mixture and injection into mice to form cartilaginous structures.

A calcium alginate mixture was obtained by combining calcium sulfate, a poorly soluble calcium salt, with a 1% sodium alginate dissolved in a 0.1 M potassium phosphate buffer solution (pH 7.4). The mixture remained in a liquid state at 4°C for 30-45 min. Chondrocytes isolated from the articular surface of calf forelimbs were added to the mixture to generate a final cellular density of 1 x 10⁷/ml (representing approximately 10% of the cellular density of human juvenile articular cartilage).

The calcium alginate-chondrocyte mixture was injected through a 22 gauge needle in 100 µl aliquots under the pannus cuniculus on the dorsum of nude mice.

The nude mice were examined 24 hours post-operatively, and all injection sites were firm to palpation without apparent diffusion of the mixture. Specimens were harvested after 12 weeks of *in vivo* incubation. On gross examination, the calcium alginate-chondrocyte specimens exhibited a pearly opalescence and were firm to palpation. The specimens weighed 0.11 ± 0.01 gms (initial weight 0.10 gms). The specimens were easily dissected free of surrounding tissue and exhibited minimal inflammatory reaction. Histologically, the specimens were stained with hematoxylin and eosin and demonstrated lacunae within a basophilic ground glass substance.

Control specimens of calcium alginate without chondrocytes had a doughy consistency 12 weeks after injection and had no histologic evidence of cartilage formation.

This study demonstrates that an injectable calcium alginate matrix can provide a three dimensional scaffold for the successful transplantation and engraftment of chondrocytes. Chondrocytes transplanted in this manner form a volume of cartilage after 12 weeks of *in vivo* incubation similar to that initially injected.

### Example 2: Effect of cell density on cartilage formation.

Varying numbers of chondrocytes isolated from the articular surface of calf forelimbs were mixed with a 1.5% sodium alginate solution to generate final cell densities of 0.0, 0.5, 1.0, and 5.0 x 10⁶ chondrocytes/ml (approximately 0.0, 0.5, 1.0, and 5.0% of the cellular density of human juvenile articular cartilage). An aliquot of the chondrocyte-alginate solution was transferred to a circular mold 9 mm in diameter and allowed to polymerize at room temperature by the diffusion of a calcium chloride solution through a semi-permeable membrane at the base of the mold. The gels formed discs measuring 2 mm in height and 9 mm in diameter.

Discs of a fixed cellular density of 5 x 10⁶ cells/ml were also formed in which the concentration of the sodium alginate and the molarity of the calcium chloride solutions were varied.

All discs were placed into dorsal subcutaneous pockets in nude mice. Samples were harvested at 8 and 12 weeks and examined for gross and histological evidence of cartilage formation.

Examinations of 8 and 12 week specimens revealed that a minimum cell density of 5 x 10⁶ chondrocytes/ml was required for cartilage production which was observed only 12 weeks after implantation. On gross examination, the specimens were discoid in shape and weighed 0.13 ± 0.01 gms (initial weight 0.125 gms). The specimens were easily dissected free of surrounding tissue and exhibited minimal inflammatory reaction. Histologically, the specimens were stained with hematoxylin and eosin and demonstrated lacunae within a basophilic ground glass substance.

Cartilage formation was independent of calcium chloride concentration used in gel polymerization. Cartilage was observed in specimens with alginate concentrations varying from 0.5% to 4.0%; however, the lowest alginate concentration tested (0.5%) showed only microscopic evidence of cartilage.

Cartilage can be grown in a subcutaneous pocket to a pre-determined disc shape using calcium alginate gel as a support matrix in 12 weeks. Cartilage formation is not inhibited by either polymerization with high calcium concentrations or the presence of high alginate concentrations but does require a minimum cellular density of 5 x 10⁶ cells/ml.

The ability to create a calcium alginate-chondrocyte gel in a given shape demonstrates that it is possible to use this technique to custom design and grow cartilaginous scaffolds for craniofacial reconstruction. Such scaffolds have the potential to replace many of the prosthetic devices currently in use.

### Example 3: Preparation of Implantable Premolded Cell-polymer mixtures.

250 µl aliquots of an isolated chondrocyte suspension was mixed with 750 µls of a 2% (w/v) sodium alginate solution (0.1 M K₂HPO₄, 0.135 M NaCl, pH 7.4). A 125 µl aliquot was placed into 9 mm diameter cell culture inserts with 0.45 µm pore size semipermeable membranes. The cell-alginate mixture was placed into contact with a 30 mM CaCl₂ bath and allowed to polymerize for 90 minutes at 37°C. After 90 minutes, the cell-alginate gel constructs were removed from the mold and had a diameter of 9 mm and a height of 2 mm. The discs were placed into the wells of 24-well tissue culture plates and incubated at 37°C in the presence of 5% CO₂ with 0.5 ml of a solution containing Hamm's F-12 culture media (Gibco, Grand Island, N.Y.) and 10% fetal calf serum (Gibco, Grand Island, N.Y.) with L-glutamine (292 µg/ml), penicillin (100 U/ml), streptomycin (100 µg/ml) and ascorbic acid (5 µg/ml) for 48 hrs.

Using this method, bovine chondrocytealginate discs were prepared, then implanted in dorsal subcutaneous pockets in athymic mice using standard sterile technique. After one, two, and three months, athymic mice were sacrificed, and the gel/chondrocyte constructs removed, weighed and placed in appropriate fixative. The cell-polymer complexes were studied by histochemical analysis.

Cartilage formation was observed histologically after three months of in vivo incubation at an initial chondrocyte density of 5 x 10⁶ cell/ml.

The above protocol was modified by using a range of CaCl₂ concentration and a range of sodium alginate concentrations. Cartilage formation was observed using 15, 20, 30, and 100 mM CaCl₂ baths and 0.5, 1.0, 1.5, 2.0, and 4.0% sodium alginate solutions.

By changing the mold within which the cell-alginate construct is created, the shape of the implant can be customized. Additionally, the mold need not be semipermeable as calcium ion can be directly mixed with the cell-alginate solution prior to being placed within a mold. The key feature is that the construct can be fashioned into a given shape prior to implantation.

### Example 4: Preparation of injectable osteoblastshydrogel mixtures.

Using the methodology described above, bovine osteoblasts have been substituted for chondrocytes and injected into animals using a hydrogel matrix.

Histology after 12 weeks of *in vivo* incubation showed the presence of early bone formation.

### Example 5: Use of the hydrogel matrix to form an immunoprotective matrix around the implanted cells.

By fashioning a cell-alginate construct as described above, one can use the hydrogel matrix to sterically isolate the encapsulated cells from the host immune system, and thereby allow allogenic cell transplants to form new tissues or organs without immunosuppression.

Bovine chondrocytes in an alginate suspension were transplanted into normal immune-competent mice. Histology after six weeks of *in vivo* incubation shows the presence of cartilage formation. Gross examination of the specimens does not demonstrate features of cartilage. Literature states that similar chondrocyte xenografts without alginate do not form cartilage.

The following examples demonstrate that human bladder muscle cell-alginate suspensions are injectable, non-migratory, and appear to conserve their volume and that autologous bladder muscle cells are useful in the endoscopic treatment of vesicoureteral reflux.

### Example 6: Isolation and Characterization of Human Bladder Muscle Cells Expanded In Vitro.

### Cell Culture:

**Human Tissue Origin.** Human bladder tissue specimens were obtained and processed within one hour after surgical removal at Children's Hospital, Boston. The specimens varied in size, ranging from one cm² to four cm². The specimens were transported to Hanks balanced salt solution containing 10 mM HEPES and 100 KIU apoprotein.

**Smooth Muscle Cells.** Muscle cells were obtained by mincing small detrusor muscle fragments to approximately 0.5 mm diameter and using these as explants in 100 mm tissue culture dishes containing 10 mL of DMEM supplemented with 10% fetal calf serum. Approximately 30 explants of similar size were added to each dish. Medium was changed twice a week. Outgrowth was routinely observed at 72 hr after explants were placed in culture. When cultures were 80% confluent, the cells were trypsinized and passaged. Cell populations highly enriched in elongated, striated cells were routinely obtained using this method. Cell strains were stained with a-actin antibody to verify their muscle phenotype.

**Results.** Populations of fusiform, striated cells could be obtained from the bladder biopsy specimens by dissection of the muscle layer followed by explant culture of the muscle fragments in DMEM supplemented with 10% calf-serum. Immunostaining with a monoclonal antibody which specifically recognized a-actin verified the muscle phenotype. These data indicate that highly proliferative populations of smooth muscle can be obtained from small biopsy specimens. The muscle cells can also be grown transiently in serum-free defined media which renders the cells free from any impurities.

### Example 7: Injection of cell suspensions into mice.

Using a 22 gauge needle, 20 nude mice were injected with a 500 microliter solution of muscle cells and alginate. Each mouse had two injection sites consisting of either control, or a solution of ten million human bladder cells per cc of alginate (32 injection sites). Injections of alginate alone or bladder muscle cells alone served as controls. Animals were sacrificed at two, four, six and eight weeks after implantation. Histologic examination of the injection areas demonstrated evidence of muscle formation in the muscle/alginate injection sites. Immunohistochemical analysis using an anti-desmin antibody indicated that the cells maintained a program of muscular differentiation. Examination of the injection sites with increasing periods of time, showed that the alginate was progressively replaced by muscle. The size of the muscle-alginate complex appeared to be uniform and stable. In both control groups (alginate alone or muscle cells alone) there were no muscle cells evident. Histologic analysis of distant organs showed no evidence of bladder muscle cells or alginate migration or granuloma formation.

### Example 8: Correction of vesicoureteral reflux in pigs using bladder muscle cells implanted in an alginate gel.

### Materials and Methods

### Animal model of vesicoureteral reflux.

The pig was used for this study because of the similarities between porcine and human bladders and kidneys. The Hanford mini-pig was used for the convenience of its smaller size. Bilateral vesicoureteral reflux was created in four mini-swine using the open bladder technique, which consists of unroofing the entire intravesical ureter, as described by Vacanti, et al., "Synthetic polymers seeded with chondrocytes provide a template for new cartilage formation" Plastic and Recon. Surg. 88:753 (1991).

Three months after the procedure, the presence of bilateral reflux was assessed by conventional radiographic cystography using an iodinated contrast agent, and by sonography using sonicated albumin, as described by Vacanti, et al., "Tissue engineered growth of new cartilage in the shape of a human ear using synthetic polymers seeded with chondrocytes" Mat. Res. Soc. Proc. 252:367 (1992). Excretory urography was performed to detect any evidence of obstruction.

*Cell Harvest.* A segment of bladder muscle was obtained from each animal. Muscle cells were harvested and plated separately in vitro. After expansion, the cells were individually quantitated and concentrated to 20 x 10⁶ cells per cc.

*Autologous bladder muscle cell-calcium alginate suspension.* Two percent weight/volume sodium alginate (0.1 M K₂PO₄, 0.135 M NaCl, pH 7.4, Protan, Portsmouth, NH) was made and sterilized in ethylene oxide. A 1.5 ml aliquot of 20 x 10⁶ cells/ml bladder muscle cell suspension was added to an equal volume of sodium alginate solution for a final alginate concentration of 1%. The bladder muscle cell-sodium alginate suspension was kept at 32°C. Immediately prior to injection, calcium sulfate (0.2 g/ml) was added to the bladder muscle cell-sodium alginate suspension. The mixture was vortexed and stored in ice until injection. The gelling process was initiated with the addition of calcium sulfate, which allowed the suspension to remain in a liquid state for approximately 40 minutes.

*Experimental study.* Mini-pigs were anesthetized with intramuscular injections of 25 ml/kg ketamine and 1 ml/kg acylpromazine. Additional anesthesia was obtained with an intramuscular administration of 25 mg/kg ketamine and 10 mg/kg of xylazine. Animals were placed in a supine position. With a 15.5 French cystoscope introduced into the bladder, a 21 gauge needle was inserted in the subureteral region of the right refluxing ureter. Approximately 2 to 3 ml of the autologous bladder muscle cell-alginate suspension (40-60 x 10⁶ bladder muscle cells) were injected through the needle, while lifting of the ureteral orifice was endoscopically visualized. The left ureteral orifice remained untreated and served as a control. Serial cystograms, cystoscopy, and excretory urographic studies were performed at eight week intervals until sacrifice. The mini-pigs were sacrificed at eight (1), 16 (1), and 26 (2) weeks after treatment. The bladder injection sites were resected and examined macroscopically and microscopically. Specimens were stained with hematoxylin and eosin, and alcian blue at a pH of 1.0 and 2.5. Histological analyses of the bladder, ureters, regional lymph nodes, kidneys, liver, and spleen were performed.

### Results

Four mini-swine underwent bilateral creation of reflux. All four were found to have bilateral reflux without evidence of obstruction at three months following the procedure. Bladder muscle cells were from each mini-swine and expanded *in vitro*. The animals then underwent endoscopic repair of reflux with the injectable autologous bladder muscle cell-alginate gel solution on the right side only.

Cystoscopic and radiographic examinations were performed at two, four, and six months after treatment. Cystoscopic examinations showed a smooth bladder wall. Cystograms showed no evidence of reflux on the treated side and persistent reflux in the uncorrected control ureter in all animals. All animals had a successful cure of reflux in the repaired ureter without evidence of hydronephrosis on excretory urography.

At the time of sacrifice, gross examination of the bladder injection site showed the presence of bladder muscle cell-alginate composites in the subureteral region. Microscopic analyses of the tissues surrounding the injection site showed no inflammation. Tissue sections from the bladder, ureters, lymph nodes, kidneys, liver and spleen showed no evidence of alginate migration or granuloma formation.

### Summary of Experimental Data

Autologous bladder muscle cells can be readily harvested, expanded *in vitro*, and injected cystoscopically. The cells survive and form a muscle nidus which is non-antigenic. This system is able to correct reflux without any evidence of obstruction.

The following examples demonstrate that chondrocyte-polymer suspensions are injectable, non-migratory, and appear to conserve their volume, and are useful in the endoscopic treatment of vesicoureteral reflux. As demonstrated in Example 1, alginate-bovine chondrocyte cell allografts were found to contain viable cartilage cells after implantation times for as long as 90 days in athymic mice. The new cartilage formed retains the approximate configuration and dimensions of the injected template. The cell-polymer construct is essential in that injection of free chondrocytes or alginate alone does not result in cartilage formation.

### Example 9: Implantation of chondrocytes in alginate gel into mice.

### Materials and Methods

Hyaline cartilage was obtained from the articular surfaces of calf shoulders and chondrocytes were harvested. Chondrocyte suspensions were concentrated to 20, 30, and 40 X 10⁶ cells per cc and mixed with dry alginate powder to form a gel. Twelve athymic mice were injected subcutaneously with a chondrocyte/alginate solution. Each mouse had four injection sites consisting of control, 10, 15, and 20 X 10⁶ chondrocyte cells (48 injection sites). Mice were sacrificed at 2, 4, 6, and 12 weeks after injection.

Histologic examination of the injection sites demonstrated evidence of cartilage formation in 34 of the 36 experimental injection sites. Gross examination of the injection sites with increasing periods of time, showed that the polymer gels were progressively replaced by cartilage. The ultimate size of the cartilage formed was related to the initial chondrocyte concentration injected and appeared to be uniform and stable within each category. There was no evidence of cartilage formation in the 12 controls. Histologic analyses of distant organs showed no evidence of cartilage or alginate gel migration or granuloma formation.

### Materials and Methods

*Animals* - Young adult athymic nu/nu mice were used as cell recipients. The animals were housed individually, allowed access to food and water as desired, and maintained on 12 hours of light and dark intervals. Anesthesia was performed with methoxyflurane by cone administration.

*Polymers* - Dry alginate impression powder (Dentsply International; Milford, DE) was used as the delivery vehicle. Alginate, a copolymer of gluronic and mannuronic acid, is designed to gel at a controlled rate when mixed with calcium salts and water. Calcium phosphates and sulfates are included in the pure polymer powder to control the gelation kinetics. The powder was sterilized in ethylene oxide and sealed in aluminum foil until injection.

*Cell Harvest -* Hyaline cartilage was obtained from the articular surfaces of calf shoulders within six hours of sacrifice. The shoulders were washed in providine-iodine 10 percent solution and chondrocytes were harvested under sterile conditions using a technique described by Klagsbrun, "Large scale preparation of chondrocytes" Methods in Enzymology, 58:560 (1979). The isolated cells were quantitated using a hemocytometer, and the chondrocyte suspension was concentrated to 20, 30, and 40 X 10⁶ cells per cc.

*Cell Delivery -* The chondrocyte cell suspensions were mixed with dry alginate powder to form a gel. Using a 21 gauge needle, 12 nude mice were injected with a 600 microliter chondrocyte/alginate solution. Each mouse had four injection sites consisting of control, 10, 15, and 20 X 10⁶chondrocytes (48 injection sites). Injection of alginate gel alone served as control in six mice. As another control six mice were injected subcutaneously in the same region with 600 microliters cell suspensions containing 10, 15, and 20 X 10⁶ chondrocytes alone, without alginate.

*Implant Recovery -* Mice were sacrificed at 2, 4, 6, and 12 weeks after injection. The implants were excised following a tissue plane that easily separated the implant from the surrounding tissue, weighted, fixed in 10 percent neutral buffered formalin, and imbedded in Paraffin. Tissue sections were also obtained from the regional lymph nodes, kidneys, bladder, ureters, lungs, spleen, and liver. Tissue sections were stained with hematoxylin and eosin. Gross and histologic examination were performed.

### Results

Figure 1a is a schematic of the general method which was used. Histologic examination of injection sites demonstrated evidence of cartilage formation in 34 of the 36 chondrocyte/alginate implants. A mild inflammatory response appeared to be resolving by four weeks. This consisted of an inflammatory response that exhibited an acute phase and a chronic foreign body reaction. Fibroblast infiltration were seen up to two weeks after injection. Examination of the injection sites with increasing periods of time, showed that the polymer gels were progressively replaced by cartilage. Gross examination showed normally appearing rubbery to hard cartilage structures. The ultimate size of the cartilage formed appeared to be related to the initial volume and chondrocyte concentration injected and appeared to be uniform within each category. The weight of the retrieved cartilage structures appeared to be stable over time. In the six polymer gel control injections (not containing chondrocytes) there was no visual evidence of cartilage formation. In the second control group (chondrocyte suspension alone) cartilage formation was not evident in any area. Histologic analysis of the peri-injection site and distant organs showed no evidence of cartilage or alginate gel migration.

### Example 10: Correction of vesicoureteral reflux in pigs using chondrocytes implanted in an alginate gel.

### Materials and Methods

### Animal model of vesicoureteral reflux.

The pig was used for this study because of the similarities between porcine and human bladders and kidneys. The Hanford mini-pig was used for the convenience of its smaller size. Bilateral vesicoureteral reflux was created in four mini-swine using the open bladder technique, which consists of unroofing the entire intravesical ureter, as described by Vacanti, et al., "Synthetic polymers seeded with chondrocytes provide a template for new cartilage formation" Plastic and Recon. Surg. 88:753 (1991).

Three months after the procedure, the presence of bilateral reflux was assessed by conventional radiographic cystography using an iodinated contrast agent, and by sonography using sonicated albumin, as described by Vacanti, et al., "Tissue engineered growth of new cartilage in the shape of a human ear using synthetic polymers seeded with chondrocytes" Mat. Res. Soc. Proc. 252:367 (1992). Excretory urography was performed to detect any evidence of obstruction.

*Cell Harvest.* Hyaline cartilage was obtained from the auricular surfaces of each mini-swine. The ears were washed with providine-iodine 10% solution and chondrocytes were harvested under sterile conditions using the technique, Atala, et al., "Endoscopic treatment of vesicoureteral reflux with a self-detachable balloon system" J. Urol. 148:724 (1992).

The isolated cells were expanded *in vitro* in a solution of Hamms F-12 media (Gibco, Grand Island, NY) with 10% fetal calf serum (Gibco), 5 micrograms/ml ascorbic acid, 292 micrograms/ml glutamine, 100 micrograms/ml streptomycin, 40 nanograms/ml vitamin D3 and 100 units/ml penicillin. The cells were incubated at 37°C in the presence of 5% CO₂. Five to eight weeks after initial harvest, the chondrocytes were trypsinized and quantitated using a hemocytometer. The chondrocyte suspension from each mini-swine was concentrated to 40 X 10⁶ cells/ml in minimal essential media - 199 (Gibco).

*Autologous chondrocyte-calcium alginate suspension.* Two percent weight/volume sodium alginate (0.1 M K₂PO₄, 0.135 M NaCl, pH 7.4, Protan, Portsmouth, NH) was made and sterilized in ethylene oxide. A 1.5 ml aliquot of 40 x 10⁶ cells/ml chondrocyte suspension was added to an equal volume of sodium alginate solution for a final alginate concentration of 1%. The chondrocyte-sodium alginate suspension was kept at 32°C. Immediately prior to injection, calcium sulfate (0.2 g/ml) was added to the chondrocyte-sodium alginate suspension. The mixture was vortexed and stored in ice until injection. The gelling process was initiated with the addition of calcium sulfate, which allowed the suspension to remain in a liquid state for approximately 40 minutes.

*Experimental study.* Mini-pigs were anesthetized with intramuscular injections of 25 ml/kg ketamine and 1 ml/kg acylpromazine. Additional anesthesia was obtained with an intramuscular administration of 25 mg/kg ketamine and 10 mg/kg of xylazine. Animals were placed in a supine position. With a 15.5 French cystoscope introduced into the bladder, a 22 gauge needle was inserted in the subureteral region of the right refluxing ureter. Approximately 2-3 ml of the autologous cartilage-alginate suspension (40-60 x 10⁶ chondrocytes) were injected through the needle, while lifting of the ureteral orifice was endoscopically visualized. The left ureteral orifice remained untreated and served as a control. Serial cystograms, cystoscopy, and excretory urographic studies were performed at eight week intervals until sacrifice. The mini-pigs were sacrificed at eight (1), 16 (1), and 26 (2) weeks after treatment. The bladder injection sites were resected and examined macroscopically and microscopically. Specimens were stained with hematoxylin and eosin, and alcian blue at a pH of 1.0 and 2.5. Histological analyses of the bladder, ureters, regional lymph nodes, kidneys, liver, and spleen were performed.

### Results

Four mini-swine underwent bilateral creation of reflux. All four were found to have bilateral reflux without evidence of obstruction at three months following the procedure. Chondrocytes were harvested from the left auricular surface of each mini-swine and expanded in vitro for 5-8 weeks, with a final concentration of 50-150 X 10⁶ viable cells per animal. The animals then underwent endoscopic repair of reflux with the injectable autologous chondrocyte-alginate gel solution on the right side only.

Cystoscopic and radiographic examinations were performed at two, four, and six months after treatment. Cystoscopic examinations showed a smooth bladder wall. Cystograms showed no evidence of reflux on the treated side and persistent reflux in the uncorrected control ureter in all animals. All animals had a successful cure of reflux in the repaired ureter without evidence of hydronephrosis on excretory urography.

At the time of sacrifice, gross examination of the bladder injection site showed a well defined rubbery to hard cartilage structure in the subureteral region. Histologic examination of these specimens using hematoxylin and eosin stains showed evidence of cartilage formation. The polymer gels were progressively replaced by cartilage with increasing time. Aldehyde fuschinalcian blue staining suggested the presence of chondroitin sulfate. Microscopic analyses of the tissues surrounding the injection site showed no inflammation. Tissue sections from the bladder, ureters, lymph nodes, kidneys, liver and spleen showed no evidence of chondrocyte or alginate migration, or granuloma formation.

### Summary of Experimental Data

Chondrocytes can be readily grown and expanded in culture. Neocartilage formation can be achieved in vitro and in vivo using chondrocyte cultured on synthetic biodegradable polymers. In these experiments, the cartilage matrix replaced the alginate as the polysaccharide polymer underwent biodegradation. Six mini-swine underwent bilateral creation of reflux. All six were found to have bilateral reflux without evidence of obstruction at three months following the procedure. Chondrocyte were harvested from the left auricular surface of each mini-swine and expanded to a final concentration of 50-150 X 10⁶ viable cells per animal. The animals then underwent endoscopic repair of reflux with the injectable autologous chondrocyte-alginate gel solution on the right side only.

Cystoscopic and radiographic examinations were performed at two, four, and six months after treatment. Cystoscopic examinations showed a smooth bladder wall. Cystograms showed no evidence of reflux on the treated side and persistent reflux in the uncorrected control ureter in all animals. All animals had a successful cure of reflux in the repaired ureter without evidence of hydronephrosis on excretory urography. The harvested ears had evidence of cartilage regrowth within one month of chondrocyte retrieval.

At the time of sacrifice, gross examination of the bladder injection site showed a well defined rubbery to hard cartilage structure in the subureteral region. Histologic examination of these specimens using hematoxylin and eosin showed evidence of normal cartilage formation. The polymer gels were progressively replaced by cartilage with increasing time. Aldehyde fuschinalcian blue staining suggested the presence of chondroitin sulfate. Microscopic analyses of the tissues surrounding the injection site showed no inflammation. Tissue sections from the bladder, ureters, lymph nodes, kidneys, lungs, liver and spleen showed no evidence of chondrocyte or alginate migration, or granuloma formation.

Accordingly, various aspects of the present invention are described in the following numbered paragraphs of the description -
1. A method for implanting tissue into an animal comprising
   mixing a biodegradable, biocompatible hydrogel solution with dissociated cells and
   implanting the mixture into the animal.
2. The method of paragraph1 wherein the hydrogel solution is hardened prior to implantation in the animal.
3. The method of paragraph1 wherein the hydrogel is injected into the animal as a cell suspension, which then hardens.
4. The method of paragraph1 wherein the hydrogel is selected from the group consisting of polysaccharides, proteins, polyphosphazines, polyethylene oxide-polypropylene glycol block copolymers, poly(acrylic acids), poly(methacrylic acids), copolymers of acrylic acid and methacrylic acid, poly(vinyl acetate), and sulfonated polymers.
5. The method of paragraph 4 wherein the polymer is selected from the group consisting of fibrin, collagen, hyaluronic acid, and other naturally occurring polymers.
6. The method of paragraph4 wherein the hydrogel is hardened by exposure to an agent selected from the group consisting of ions, pH changes, and temperature changes.
7. The method of paragraph6 wherein the hydrogel is hardened by interaction with ions selected from the group consisting of cations selected from the group consisting of copper, calcium, aluminum, magnesium, strontium, barium, tin, and di-, tri- or tetra-functional organic cations; anions selected from the group consisting of low molecular weight dicarboxylic acids, sulfate ions and carbonate ions.
8. The method of paragraph4 wherein the hydrogel is further stabilized by cross-linking with a polyion.
9. The method of paragraph1 wherein the cells are selected from the group consisting of chondrocytes and other cells that form cartilage, osteoblasts and other cells that form bone, muscle cells, fibroblasts, and organ cells.
10. The method of paragraph1 wherein the hydrogel is molded to form a specific shape prior to implantation.
11. The method of paragraph1 wherein the hydrogel is molded to form a specific shape after mixing with the cells and being implanted into the animal.
12. The method of paragraph1 wherein the defect is vesicoureteral reflux.
13. The method of paragraph1 wherein the patient has incontinence.
14. The method of paragraph1 wherein the defect is in the thoracic region.
15. The method of paragraph1 wherein the defect is in the upper gastrointestinal tract.
16. A composition for implanting tissue into an animal comprising
   a hydrogel solution mixed with dissociated cells.
17. The composition of paragraph16 wherein the hydrogel solution is hardened prior to implantation in the animal.
18. The composition of paragraph16 wherein the hydrogel is injected into the animal as a cell suspension, which then hardens.
19. The composition of paragraph16 wherein the hydrogel is selected from the group consisting of polysaccharides, proteins, polyphosphazines, polyethylene oxide-polypropylene glycol block copolymers, poly(acrylic acids), poly(methacrylic acids), copolymers of acrylic acid and methacrylic acid, poly(vinyl acetate), and sulfonated polymers.
20. The suspension of paragraph 19 wherein the polymer is selected from the group consisting of fibrin, collagen, hyaluronic acid, and other naturally occurring polymers.
21. The composition of paragraph 18 wherein the hydrogel is hardened by exposure to an agent selected from the group consisting of ions, pH changes, and temperature changes.
22. The composition of paragraph 21 wherein the hydrogel is hardened by interaction with ions selected from the group consisting of cations selected from the group consisting of copper, calcium, aluminum, magnesium, strontium, barium, tin, and di-, tri- or tetra-functional organic cations; anions selected from the group consisting of low molecular weight dicarboxylic acids, sulfate ions and carbonate ions.
23. The composition of paragraph22 wherein the hydrogel is further stabilized by cross-linking with a polyion.
24. The composition of paragraph 16 wherein the cells are selected from the group consisting of chondrocytes and other cells that form cartilage, osteoblasts and other cells that form bone, muscle cells, fibroblasts, and organ cells.

Modifications and variations of the compositions and methods of the present invention will be obvious to those skilled in the art from the foregoing detailed description. Such modifications and variations are intended to come within the scope of the following claims.

## Claims

1. Use of a mixture of a solution of a biodegradable, biocompatible natural or synthetic organic polymer, and dissociated cells;
wherein the polymer is capable of being cross-linked via covalent, ionic, or hydrogen bonds to create a three-dimensional open-lattice structure which entraps water molecules to form a gel, and
wherein the dissociated cells are selected from the group consisting of cells that form cartilage, cells that form bone, muscle cells, fibroblasts, and cells acting primarily to synthesize, secrete or metabolize materials;
in the manufacture of a cell-polymeric solution having between 1 and 50 million of said cells per millilitre dispersed therein, for forming tissue within an animal by injection into the animal,
wherein the solution cross-links to form a polymeric hydrogel having cells dispersed therein inside the animal, whereby the cells form tissue.

2. A mixture of a solution of a biodegradable, biocompatible natural or synthetic organic polymer, and dissociated cells;
wherein the polymer is capable of being cross-linked via covalent, ionic, or hydrogen bonds to create a three-dimensional open-lattice structure which entraps water molecules to form a gel, and
wherein the dissociated cells are selected from the group consisting of cells that form cartilage, cells that form bone, muscle cells, fibroblasts, and cells acting primarily to synthesize, secrete or metabolize materials; and
wherein the mixture forms an injectable cell-polymeric solution having between 1 and 50 million of said cells per millilitre dispersed therein;
for use in forming tissue within an animal by injection into the animal, wherein the solution cross-links to form a polymeric hydrogel having cells dispersed therein inside the animal, whereby the cells form tissue.

3. The use or mixture of claim 1 or 2 wherein the cell-polymeric solution has between 10 and 20 million cells per millilitre.

4. The use or mixture of any one of claims 1 to 3 wherein the cross-linking of the polymer solution is initiated prior to implantation in the animal.

5. The use or mixture of any one of claims 1 to 3 wherein the cross-linking of the polymer solution is initiated after the polymer solution is injected into the animal.

6. The use or mixture of any one of claims 1 to 5 wherein the polymer is selected from the group consisting of alginate, polyphosphazines, polyethylene-oxide-polypropylene glycol block copolymers, poly(acrylic acids), poly(methacrylic acids), poly(vinyl acetate), and sulfonated polymers.

7. The use or mixture of claim 6 wherein the polymer solution is cross-linked by using ions, altering the pH or changing the temperature.

8. The use or mixture of any one of claims 1 to 7 wherein the polymer solution is cross-linked by interaction with cations selected from the group consisting of copper, calcium, aluminum, magnesium, strontium, barium, tin, and di-, tri- and tetrafunctional organic cations; or anions selected from the group consisting of low molecular weight dicarboxylic acids, sulfate ions and carbonate ions.

9. The use or mixture of claim 6 wherein the hydrogel is further stabilized by cross-linking with a polyion added to the cell-polymeric solution before injection.

10. The use or mixture of any one of claims 1 to 9 wherein the polymer is a synthetic polymer.

11. A kit for formation of tissue at a site in a patient comprising
a cell-polymeric solution comprising a biodegradable, biocompatible natural or synthetic organic polymer, wherein the polymer cross-links after injection via covalent, ionic, or hydrogen bonds to create a polymeric hydrogel having dispersed therein between 1 and 50 million dissociated heterologous cells per millilitre, wherein the cells are selected from the group consisting of cells that form cartilage, cells that form bone, muscle cells, fibroblasts, and cells acting primarily to synthesize, secrete or metabolize materials, the hydrogel consisting of the injected cell-polymeric solution, suitable for implantation into a patient to form tissue
in combination with means for injection of the cell-polymeric solution into the site in the patient where tissue is to be formed.

12. The kit of claim 11 wherein the polymer is a synthetic polymer.

13. The kit of claim 11 or 12 wherein the polymeric hydrogel has between 10 and 20 million dissociated heterologous cells per millilitre dispersed therein.
